# EUROPÄISCHE PATENTSCHRIFT

(11) **EP 1 536 848 B1**
(45) Veröffentlichungstag und Bekanntmachung des Hinweises auf die Patenterteilung: **14.11.2007**
(21) Anmeldenummer: 03750507.0
(22) Anmeldetag: 10.09.2003
(51) Int. Cl.: A61L 29/16

(54) **VERFAHREN ZUR HERSTELLUNG EINES ANTIMIKROBIELLEN KUNSTSTOFFPRODUKTES**
METHODS FOR PRODUCING AN ANTI-MICROBIAL PLASTIC PRODUCT
PROCEDE DE FABRICATION D'UN PRODUIT ANTIMICROBIEN EN MATIERE PLASTIQUE

(30) Priorität: 10.09.2002 DE 10241962; 10.07.2003 DE 10331324
(43) Veröffentlichungstag der Anmeldung: 08.06.2005
(73) Patentinhaber: Prof. Dr. Josef-Peter Guggenbichler,Dr. Christoph Cichos GBR Antimicrobial Argentum Technologies, 80336 München (DE)
(72) Erfinder: GUGGENBICHLER, Josef-Peter, 80336 München (DE); CICHOS, Christoph, 09599 Freiberg (DE)
(74) Vertreter: König, Gregor Sebastian
(86) Internationale Anmeldenummer: PCT/EP2003/010049
(87) Internationale Veröffentlichungsnummer: WO 2004/024205

(56) Entgegenhaltungen:
- EP-A- 1 375 700
- WO-A-01/24839
- WO-A-01/43788
- WO-A-94/04202
- WO-A-98/31404
- DE-A- 19 936 059

## Beschreibung

Die Erfindung betrifft Verfahren zur Herstellung metallhaltiger antimikrobieller Kunststoffprodukte sowie durch das Verfahren erhältliche Produkte, insbesondere Produkte für den medizinischen Bedarf.

Kunststoffgegenstände werden im medizinischen Bereich sehr häufig und für verschiedenste Zwecke verwendet. Problematisch bei der Verwendung von Kunststoffprodukten für medizinische Zwecke ist die leichte Besiedelbarkeit der Kunststoffe mit Keimen. Die Keime setzen sich auf der Kunststoffoberfläche fest und bilden einen "Biofilm". Die Folge der Verwendung eines mit Mikroorganismen besiedelten Kunststoffgegenstandes sind oft Infektionen. Es ist bekannt, dass die Verwendung von Kathetern und Kanülen aus Kunststoffen leicht zu einer Infektion durch Einwanderung von Bakterien führen kann. Besonders gravierend und häufig sind solche Infektionen u.a. bei zentralvenösen Kurz-, Mittel- und Langzeitkathetern sowie im urologischen Bereich, wo Harnröhrenkatheter und Ureterenkatheter routinemäßig verwendet werden und bei ableitenden Ventikelsystemen. So sterben alleine in der Bundesrepublik Deutschland täglich ungefähr 12 bis 15 Patienten infolge von Infektionen, die auf die Verwendung von mikrobiell verunreinigten Kathetern zurückzuführen sind.

Bislang wurden zahlreiche Versuche unternommen, die Besiedelung von Kunststoffgegenständen und somit Infektionen zu verhindern. WO87/03495 und WO89/04682 beschreiben die Imprägnierung von medizinischen Vorrichtungen bzw. Implantaten mit Antibiotika. Problematisch bei der Imprägnierung mit Antibiotika ist allerdings die Bildung und Selektion von resistenten Mikroorganismen.

Ein weiterer Ansatz zur Verringerung von Infektionen bei der Verwendung von Kunststoffprodukten ist die Verwendung von Metallen oder Metalllegierungen, z.B. bei Kathetern (DE 40 41 721, DE 27 20 776 und DE 33 02 567). Von besonderer Bedeutung ist hierbei die antimikrobielle Eigenschaft von Silber. Bereits Spuren von Silber und seinen Salzen zeigen eine bakteriostatische und bakterizide Wirkung. US 4 054 139 offenbart einen Katheter, bei dem zur Infektionsprophylaxe ein silberhaltiges, oligodynamisches Material auf innere und äußere Oberflächen appliziert wurde. Allerdings gelang es in den beschriebenen Ansätzen bislang nicht, in jeder Hinsicht, insbesondere bei Beginn der Benutzung zufriedenstellende Ergebnisse hinsichtlich der Sterilität mit der Imprägnierung von Kunststoffprodukten zu erzielen.

Ein Verfahren zur Herstellung von antimikrobiellen Kunststoffkörpern mit verbessertem Langzeitverhalten wird in WO01/09229 beschrieben.

In einer klinischen Prüfung der in WO 01/09229 beschriebenen Katheter konnte eine Reduktion septischer Komplikationen um 88 % gegenüber den durch herkömmliche Katheter verursachten Infektionen beobachtet werden. Das bedeutet, dass im Vergleich zur Verwendung der Kontrollkatheter, wobei 25 Fälle von Sepsis auftraten, die Sepsisfälle auf drei Fälle verringert werden konnten. Somit ist die Wirkung eines nach dem in WO 01 /09229 offenbarten Verfahren hergestellten Katheters zwar gegenüber dem bisherigen Stand der Technik deutlich verbessert, aber auch bei der Verwendung der in WO 01/09229 offenbarten Katheter wird eine Besiedelungsrate von 10 % beobachtet, zudem treten auch hier, insbesondere in den ersten Tagen nach der Implantation des Katheters Infektionen an der Eintrittsstelle des Katheters auf.

Somit ist es bislang nicht gelungen, eine mikrobielle Verunreinigung von medizinisch verwendeten Kunststoffprodukten, insbesondere von Kathetern in zufriedenstellendem Ausmaß zu verhindern.

Die Aufgabe der vorliegenden Erfindung ist somit die Bereitstellung eines Verfahrens zur Herstellung von Kunststoffprodukten, die eine zufriedenstellende antimikrobielle Wirksamkeit aufweisen.

Diese Aufgabe wird gelöst durch ein Verfahren zur Herstellung eines antimikrobiellen Kunststoffproduktes, umfassend
A) Bilden eines Vorprodukts,
B) Behandeln von mindestens einem Bestandteil des Vorproduktes mit einem antimikrobiellen kolloiden Metalls, und
C) Zusetzen eines löslichen oder schwerlöslichen Salzes eines antimikrobiellen Metalls und
D) Formen des Kunststoffproduktes.

Überraschenderweise ergibt die Kombination aus einem antimikrobiellen kolloiden Metalls und einem löslichen, bevorzugt schwerlöslichen Salz eines antimikrobiellen Metalls eine zufriedenstellende antimikrobielle Wirksamkeit. Neben einer ausreichenden Langzeitwirkung wird mit den erfindungsgemäßen Kunststoffprodukten auch eine deutlich verbesserte Sofortwirkung gegenüber Mikroorganismen erreicht. Insbesondere ist die antimikrobielle Wirksamkeit zu Beginn wesentlich verbessert gegenüber einem Kunststoffprodukt des Standes der Technik, wie beispielsweise in WO 01/09229 beschrieben. So kann im direkten Vergleich der Kunststoffprodukte hergestellt gemäß WO 01 /09229 und der erfindungsgemäßen Kunststoffprodukte eine deutlich höhere antimikrobielle Wirksamkeit der erfindungsgemäßen Kunststoffprodukte gezeigt werden (vgl. Tabelle 1).

Die Kunststoffprodukte gemäß der vorliegenden Erfindung besitzen zudem keine erhöhte Cytotoxizität gegenüber Produkten des Standes der Technik, ein weiterer Vorteil ist es, dass bei der Verwendung der erfindungsgemäßen Kunststoffprodukte keine Thrombogenität beobachtet wird.

Antimikrobielle Kunststoffprodukte im Sinne der Erfindung sind Produkte, die eine Wirksamkeit gegen Mikroorganismen aufweisen, insbesondere gegen Bakterien oder/und Pilze. Dabei kann es sich sowohl um eine bakteriostatische Wirkung als auch um eine bakterizide Wirkung handeln.

Durch das erfindungsgemäße Verfahren kann prinzipiell jedes beliebige antimikrobielle Kunststoffprodukt hergestellt werden, bevorzugt werden Produkte hergestellt, die im medizinischen Bereich Verwendung finden. Dabei kann es sich beispielsweise um Katheter, Schläuche, Tuben, insbesondere endotracheale Tuben, in der Urologie verwendete Gegenstände, Knochenzement, bevorzugt Knochenzement, der aus Methylacrylat besteht, Goretexgewebe, Zahnbürsten, Silikonkunststoffe, Kunststofffolien, Textilien, beispielsweise zur Herstellung von Berufskleidung, Windeln oder/und Teile davon handeln. In einer besonders bevorzugten Ausführungsform des erfindungsgemäßen Verfahrens werden Katheter hergestellt.

Als Ausgangsmaterialien zur Herstellung der erfindungsgemäßen antimikrobiellen Kunststoffprodukte können beliebige polymere Verbindungen eingesetzt werden, die im medizinischen Bereich üblicherweise Verwendung finden. Bevorzugte Polymere sind z.B. Polyurethane, Polyethylen, Polypropylen, vernetzte Polysiloxane, Polymere auf (Meth)acrylat-Basis, Cellulose und Cellulose-Derivate, Polycarbonate, ABS, Tetrafluorethylenpolymere, Polyethylenterephthalate sowie die entsprechenden Copolymere. Besonders bevorzugt werden Polyurethan, Polyethylen und Polypropylen sowie Polyethylen/Polypropylen-Copolymere verwendet, am meisten bevorzugt ist Polyurethan.

Neben einem oder mehreren polymeren Materialien kann das Vorprodukt weitere Additive umfassen. Additive können beispielsweise anorganische oder organische Substanzen sein. Das Vorprodukt kann alle anorganischen als auch organischen Substanzen umfassen, die inert und medizinisch unbedenklich sind, so wie beispielsweise Bariumsulfat, Calciumsulfat, Strontiumsulfat, Titandioxid, Aluminiumoxid, Silicumdioxid, Zeolithe, Calciumfluorid (CaF₂), Glimmer, Talk, pyrogene Kieselsäure, Calciumhydroxylapatit, Kaolin, Zirkon oder/und Mikrocellulose. Bevorzugt verwendete anorganische Substanzen sind Bariumsulfat, welches gleichzeitig als Röntgenkontrastmittel für besondere Anwendungsformen eingesetzt werden kann, und Zirkon.

Im erfindungsgemäßen Verfahren werden ein oder mehrere Bestandteile des Vorproduktes mit einem kolloiden Metall behandelt. Hierbei können ein oder mehrere polymere Materialien oder/und ein oder mehrere anorganische bzw. organische Teilchen mit dem kolloiden Metall behandelt werden. Die Trägermaterialien für das kolloide Metall können im Vorprodukt in einer Menge von etwa 5 bis 50 Gew.-% vorliegen. Wenn Bariumsulfat als Trägermaterial eingesetzt wird, liegt es gebräuchlicherweise in einer Menge von etwa 5 bis 30 Gew.-% vor, besonders bevorzugt in einer Menge von etwa 20 Gew.-%. Bei der Verwendung von Siliciumdioxid als Trägermaterial liegt dieses in einer Menge von etwa 30 bis 50 Gew.-%, bevorzugt etwa 40 Gew.-% vor.

Das kolloide Metall, mit dem ein oder mehrere Bestandteile des Vorprodukts behandelt werden, wird geeigneterweise durch Reduktion von Metallsalzlösungen hergestellt. Bei der Verwendung von Silber wird dieses beispielsweise als ammonialkalische Silbernitratlösung mit einem Reduktionsmittel versetzt. Zur Stabilisierung des entstehenden Metallkolloids können gegebenenfalls zusätzlich Schutzstoffe wie Gelatine, Kieselsäure, Stärke, Dextrin, Gummi Arabicum, Polyvinylalkohol oder Komplexbildner wie Ethylendiamintetraessigsäure eingesetzt werden. Vorzugsweise wird ohne Schutzstoffe gearbeitet. Geeignete Reduktionsmittel sind beispielsweise Aldehyde (z.B. Acetaldehyd), Aldosen (z.B. Glucose), Chinone (z.B. Hydrochinon), anorganische komplexe Hydride (Natrium- oder Kaliumboranat), reduzierende Stickstoffverbindungen (z.B. Hydrazin, Polyethylenimin), Ascorbinsäure, Weinsäure sowie Zitronensäure.

Durch Variation der Reduktionsmittel sowie Variation oder Weglassen der Stabilisatoren kann zudem die Färbung des beschichteten Trägermaterials gesteuert werden.

Für das erfindungsgemäße Verfahren sind alle antimikrobiell wirkenden Metalle, so wie beispielsweise Silber, Kupfer, Gold, Zink, Zirkonium, Wismut (Bismut) oder Cer sowie Gemische davon geeignet. Besonders bevorzugt ist Silber, welches eine hohe antimikrobielle Wirksamkeit aufweist. Weiterhin wird bevorzugt Kupfer verwendet, wodurch vorteilhafterweise auch eine Wirksamkeit gegenüber Pilzen erreicht wird.

Die Menge des kolloiden Metalls beträgt vorteilhafterweise etwa 0,1 bis 10, bevorzugt etwa 0,5 bis 5 Gew.-%.

Das Aufbringen des kolloiden Metalls auf einen oder mehrere Bestandteile des Vorprodukts kann entweder in einem Schritt erfolgen oder kann von einer Trocknung gefolgt werden und mehrmals wiederholt werden. Mit beiden Methoden kann eine sehr hohe Metallkonzentration erreicht werden. Durch Variation der Reduktionsmittel sowie Variation oder Weglassen der Stabilisatoren kann die Partikelgröße des Metalls gesteuert werden. Wenn Silber als Metallkolloid verwendet wird, liegt die bevorzugte Teilchengröße im Bereich von 10 bis 50 nm. Silber dieser Teilchengröße wird als Nanosilber bezeichnet. In einer bevorzugten Ausführungsform wird nach der Zugabe des Reduktionsmittels und der Abscheidung des Nanosilbers in der Lösung verbliebenes Silber durch Zugabe von Phosphorsäure als Silberphosphat gefällt, welches nachstehend als "Silberphosphat in statu nascendi" bezeichnet wird und sich durch besonders raschen Eintritt der antimikrobiellen Wirkung auszeichnet.

Die Menge des kolloiden Metalls wird so gewählt, dass ein ausreichender Teil der Oberfläche des Kunststoffproduktes aus Metallteilchen besteht, um eine antimikrobielle Wirksamkeit zu erreichen.

Erfindungsgemäß wird dem Vorprodukt weiterhin ein lösliches oder schwerlösliches Salz eines antimikrobiellen Metalls zugesetzt. Hierbei handelt es sich bevorzugt um ein Silbersalz, Zinksalz, Kupfersalz, Cersalz, Platinsalz, Zirkoniumsalz, Wismutsalz oder/und Goldsalz sowie Gemische davon. Besonders bevorzugt wird ein Silbersalz, insbesondere Silbersulfat oder/und Silberphosphat in statu nascendi, verwendet. Grundsätzlich sind alle löslichen oder schwerlöslichen Salze antimikrobiell wirksamer Metalle geeignet, die gegenüber Lichteinwirkung beständig und physiologisch unbedenklich sind. Die Menge des verwendeten Metallsalzes kann von 0,1 bis 5 Gew.-% bezogen auf das Gesamtgewicht des Vorproduktes betragen, bevorzugt von 0,5 bis 1 Gew.-%.

Nach dem Mischen der zumindest teilweise mit einem Metallkolloid behandelten Bestandteile des Vorproduktes mit dem schwerlöslichen Metallsalz wird das erhaltene Gemisch weiterverarbeitet, um ein Kunststoffprodukt zu erhalten. Dies kann beispielsweise durch Extrudieren, Spritzgießen, Mischen, Kneten oder (Heiß-)Pressen erfolgen. Bevorzugte Formungsverfahren sind Extrudieren sowie Spritzgießen.

Ein weiterer Gegenstand der vorliegenden Erfindung sind Kunststoffprodukte, die durch das erfindungsgemäße Verfahren erhältlich sind. Bevorzugt handelt es sich um Kunststoffprodukte, die im medizinischen Bereich Verwendung finden. In einer besonders bevorzugten Ausführungsform werden durch das erfindungsgemäße Verfahren Katheter hergestellt.

Beispiele für die bevorzugten Medizinprodukte sind Venenkatheter zur Kurzzeitimplantation, bei denen sowohl die Außenseite des Katheters als auch jedes Lumen innen, der Luer Lock und der Verteiler aus dem erfindungsgemäß erhaltenen Material bestehen. Versuche haben gezeigt, dass eine Inokulumgröße von 10⁹ Keimen, mit denen die Oberfläche kontaminiert wurde, innerhalb von weniger als 9 Stunden vollständig eliminiert wird. Weiterhin sind periphere Venenkanülen, Sheldon-Katheter zur Implantation über 6 Wochen zur Hämodialyse, Hickman-Typ-Katheter zur Langzeitimplantation mit einem aus erfindungsgemäß hergestellten Material bestehenden Cuff (Antimikrobielle Wirksamkeit mindestens 1 Jahr festgestellt), Portkatheter, wobei mindestens die Portkammer aus erfindungsgemäß hergestelltem Material besteht, zweckmäßig auch alle weiteren Bestandteile desselben, ableitende Ventrikelkatheter (minimale Wirksamkeitsdauer 3 Jahre), Blasenkatheter, Cystostomie, Nephrostomiekatheter, Uretherstents (z.B. aus Polyuretahn- oder Silikongrundmaterial; zweckmäßig besteht auch das gesamte Harnsammelsystem und die Konnektoren aus diesem Material), Thoraxdrainagen sowie das angeschlossene Saugsystem, endotracheale Tuben, Tenckhof-Katheter mit Cuff, Knochenzemente (z.B. auf Basis von Methylacrylat), Zahnbürsten (Borsten und Griff), chirurgisches Nahtmaterial, Fadenmaterial zur Herstellung von antimikrobiellen Textilien, Lacke zur antimikrobiellen Beschichtung, z.B. von Schläuchen für Beatmung, antimikrobielle Wundabdeckungen und Dressings bei Brandverletzungen.

Im Folgenden werden bevorzugte Ausführungsformen des erfindungsgemäßen Verfahrens beschrieben.

In einer bevorzugten Ausführungsform werden als polymeres Material Polyurethanpellets mit einer Größe von etwa 1 mm³ verwendet. Ein weiterer Bestandteil des Vorprodukts ist Bariumsulfat, welches als Trägermaterial fungiert. Auf dem Bariumsulfat sind etwa 3 bis 10 Gew.-%, gegebenenfalls auch mehr Nanosilber abgeschieden. Außerdem umfasst das Vorprodukt etwa 0,5 bis 1 Gew.-% Silbersulfat oder Silberphosphat, insbesondere in statu nascendi. Die Bestandteile des Vorproduktes werden gemischt, die weitere Verarbeitung kann durch Extrudieren erfolgen.

In einer weiteren bevorzugten Ausführungsform wird als Metallsalz eine Kombination aus Silber und Kupfer in einem Silber/Kupfer-Verhältnis von etwa 2:1 verwendet. Diese Kombination besitzt vorteilhafterweise auch eine zufriedenstellende mikrobielle Wirksamkeit gegenüber Pilzen.

Gemäß einer weiteren bevorzugten Ausführungsform wird eine Kombination aus einem kolloiden Metall, besonders bevorzugt Nanosilber, und Zirkonsilikat verwendet. Besonders geeignet sind Gewichtsverhältnisse Silber zu Zirkonsilikat von 1:1-10.

Die Erfindung wird weiterhin durch die nachfolgenden Figuren und Beispiele veranschaulicht.

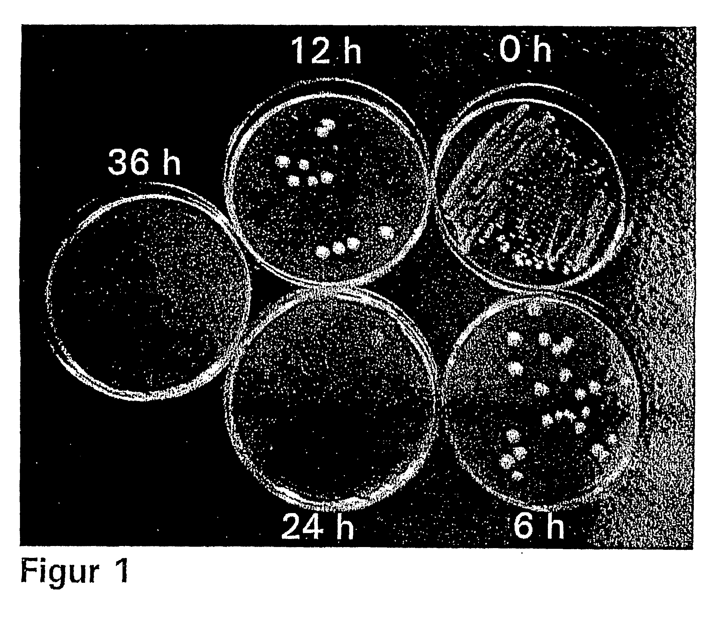

Die Figuren 1 bis 3 zeigen Ergebnisse von Versuchen zur antimikrobiellen Wirksamkeit. Als Mikroorganismus wurde jeweils Staphylokokkus epidermidis ATCC 14 990 mit einer Ausgangskeimzahl von 5 x 10⁷ CFU/ml verwendet.

In dem in Figur 1 gezeigten Versuchsansatz wurden 0,8 % Nanosilber und 0,5 % Silbersulfat verwendet.

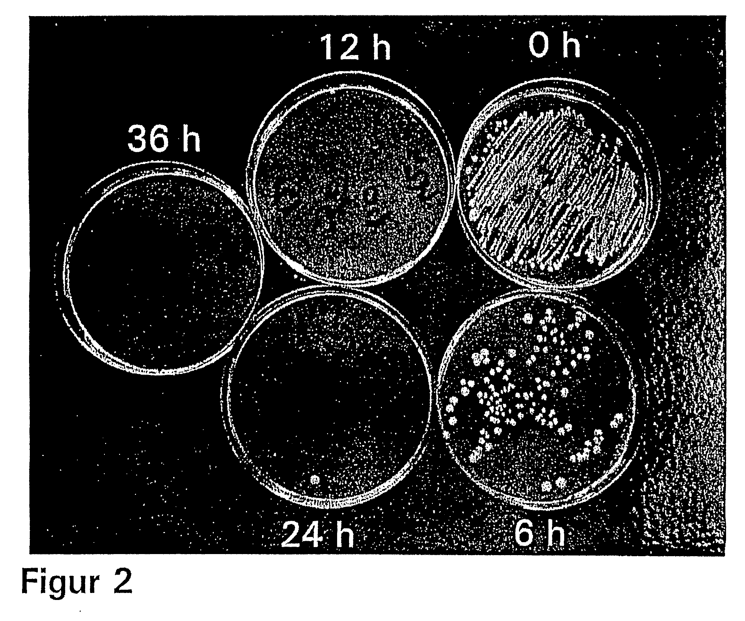

In dem in Figur 2 gezeigten Versuchsansatz wurden 0,8 % Nanosilber und 1,0 % Silbersulfat eingesetzt.

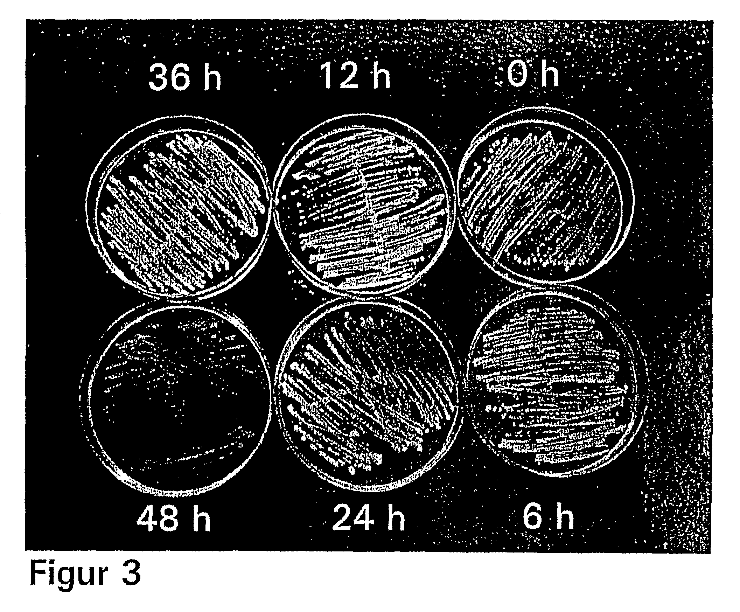

Figur 3 zeigt einen Versuchsansatz, worin 0,8 % Nanosilber und kein zusätzliches Silbersulfat eingesetzt wurde.

### Beispiele:

**Vergleichsbeispiel 1: Handelsüblicher Kunststoff gemäß WO 01/09299**

### A: Herstellung eines Silberkolloids

In 100 ml destilliertem Wasser werden 1,0 g (5,88 mmol) AgNO₃ p.a. gelöst, die Lösung wird mit 1,0 ml (14,71 mmol) 25 %-igem NH₃-Wasser versetzt. Zu dieser Lösung wird zur Darstellung des Silberkolloids bei 40 °C über einen Zeitraum von 30 Minuten langsam eine Lösung aus 258,7 mg (5,88 mmol, 330µl) Acetaldehyd in 50 ml aqua dest. getropft.

### B: Beschichtung von Polyurethanpellets

10 Minuten nach dem Beenden des Zutropfens, wie in Beispiel 1 beschrieben, werden ca. 50 g Polyurethanpellets aus Tecothane TT-1085A zugesetzt und zur Beschichtung mit kolloidalem Silber zunächst für 2 Stunden bei 40 °C und anschließend für 3 Stunden bei Raumtemperatur kräftig gerührt. Das Silberkolloid wird durch rasche Filtration über einen Faltenfilter mit geeigneter Porengröße abgetrennt, die Pellets werden noch einmal mit dem Filtrat nachgewaschen und die noch feuchten Pellets werden in eine Abdampfschale überführt. Nach dem Enfernen überschüssiger nicht am Polymer haftender Silberkolloidlösung erfolgt eine Trocknung für 10 Stunden bei 70°C.

### Beispiel 2: Kunststoff mit verbesserterer antimikrobieller Wirksamkeit.

### A: Adsorption von kolloidalem Silber an Bariumsulfat

In 360 ml destilliertem Wasser werden bei 50 °C nacheinander gelöst: 0,6 g Gelatine und 6,0 g AgNO₃. Die Lösung wird mit 7,8 ml 25 %-iger wässriger Ammoniaklösung versetzt. Unter kräftigem Rühren wird bei 50 °C langsam eine Lösung aus 3,18 g wasserfreier Glucose, in 120 ml destilliertem Wasser gelöst, zudosiert. Wenn etwa die Hälfte der Glucosemenge zugetropft wurde, werden in das bereits gebildete Silberkolloid unter starkem Rühren 100 g Bariumsulfat eingetragen, und die Glucosedosierung wird fortgesetzt. Nach Beendigung der Glucosezugabe wird die Suspension noch für weitere 2 Stunden zunächst bei 50 °C und danach noch 3 Stunden bei 70 °C turbiniert.

Anschließend wird der Feststoff durch Filtration oder Zentrifugation von der Flüssigkeit getrennt. Der Feststoff wird mehrfach bis zur Elektrolytfreiheit mit Reinstwasser gewaschen, filtriert, bei 70 °C bis 80 °C getrocknet und fein zerkleinert.

### B: Zumischen von Silbersulfat

Dem getrockneten und zerkleinertem Bariumsulfat werden 2,5 Gew.-% bzw. 5 Gew.-% fein aufgemahlenes Silbersulfat zugefügt und intensiv vermischt.

### C: Mischen der einzelnen Bestandteile

20 Gew.-% des beschichteten Bariumsulaft/Silbersulfatgemisches werden mit 77,6 Gew.-% Polyurethanpellets und 2,4 Gew.-% eines weiteren, anorganischen unbeschichteten Materials, z.B. Titandioxid, gründlich gemischt und das Gemisch wird einer weiteren Bearbeitung, z.B. einer Extrusion unterzogen.

Werden in Schritt B 2,5 Gew.-% Silbersulfat zugesetzt, erhält man den in Tabelle 1 unter A aufgeführten Kunststoff, werden in Schritt B 5 Gew.-% Silbersulfat zugegeben, wird der in Tabelle 1 unter B aufgeführte Kunststoff erhalten.

### Beispiel 3: Kunststoff mit verbesserter antimikrobieller Wirksamkeit

### A: Adsorption von kolloidalem Silber an Bariumsulfat

In 1080 ml destilliertem Wasser werden bei 50 °C 18 g AgNO₃ gelöst und 200 g Bariumsulfat zugesetzt. Die Suspension wird für ca. 20 Minuten kräftig gerührt und danach mit 23,4 ml einer 25 %-igen wässrigen Ammoniaklösung versetzt.

Unter ständigem Rühren werden bei gleichbleibender Tempratur 9,6 g wasserfreie Glucose in 360 ml gelöst langsam zugetropft. Nach Beendigung der Glucosedosierung wird analog Beispiel 2A bis zur Mahlung des getrockneten Bariumsulfates weiterverfahren.

### B: Zumischen von Silbersulfat

Die Zumischung von Silbersulfat erfolgt analog Beispiel 2 B.

### C: Mischen der einzelnen Bestandteile

Analog Beispiel 2 wird das mit Silbersulfat vermischte Bariumsulfat mit den anderen Bestandteilen gemischt und weiterverarbeitet.

### Beispiel 4: Bestimmung der antimikrobiellen Wirksamkeit

Zur Bestimmung der antimikrobiellen Wirksamkeit der erfindungsgemäßen Kunststoffe wurden Proben der entsprechenden Kunststoffe mit einer verschiedene Keime enthaltenden Trypcase-Soy-Broth-Nährlösung bei 37 °C inkubiert.

### Verwendete Mikroorganismen:

Staphylokokkus epidermidis (S. epidermidis) ATCC 14 990,
S. epidermidis, frisches klinisches Isolat von einem Patienten mit einer Katheter-assoziierten Sepsis,
Staphylokokkus aureus (S. aureus) ATCC 25923,
Escherichia coli (E.coli), frisches klinisches Isolat von einem Patienten mit einer Katheter-assoziierten Sepsis,
Pseudomonas aeruginosa (P. aeruginosa), frisches klinisches Isolat von einem Patienten mit einer Katheter-assoziierten Sepsis.

Die Keimzahl wurde entweder auf 5 x 10⁷ colony forming units (CFU)/ml (entspricht bei Staphylokokken einer OD von 0,30 bei 457 nm, bei P. aeruginosa und E. coli einer OD von 0,65) oder 10⁹ CFU/ml (OD 0,65 für Staphylokokken bei 475 nm, 1,2 für P. aeruginosa und E. coli) im Fotometer eingestellt. Parallel wurde eine Bestimmung der CFU/ml durch Serienverdünnung auf Agarplatten durchgeführt und die durch fotometrische Messung ermittelten Keimzahlen bestätigt.

### Kunststoffmaterialien:

Es wurde Polyurethan (Tecoflex) verwendet, ein Material, aus dem praktisch alle implantierbaren zentralvenösen Katheter gefertigt werden. Dieses wurde mit Nanosilber (Partikelgröße 3 bis 5 nm) in einer Menge von 0,8 bzw. 1,3 Gew.-% und unterschiedlichen. Konzentrationen von Silbersulfat (0,25, 0,5, 0,75 und 1,0 %) coextrudiert. Es wurden Stränge mit einem Außendurchmesser von 1,6 mm gefertigt. Davon wurden Pellets von jeweils 1 mm Länge abgeschnitten, 10 Pellets ergeben eine Oberfläche von etwa 1 cm² bzw. 50 Pellets ergeben eine Oberfläche von 5 cm².

### Testmethode:

Die Kunststoffstückchen (entweder mit einer Oberfläche von 1 cm² oder 5 cm⁵) wurden in eine Suspension mit entweder 5 x 10⁷ CFU/ml oder 10⁹ CFU/ml der oben beschriebenen Keime in physiologischer Kochsalzlösung eingebracht. Die Eprouvetten wurden mit einer Geschwindigkeit von 120 Rotationen/Minute geschüttelt. Zu Beginn der Untersuchung (Ausgangskeimzahl) und nach 6, 12, (18), 24, 36 und (48) Stunden wurde jeweils 1 Öse (2 µl) entnommen und auf eine Agarplatte (Müller Hinton Agar) ausgestrichen. Die Platten wurden bei 37 °C für 24 Stunden bebrütet. Anschließend wurde die Keimzahl auf der Agarplatte durch Zählung der Kolonien bestimmt.

Alle Versuche wurden dreifach wiederholt, die folgenden Daten stellen jeweils die Mittelwerte der drei korrespondierenden Versuchsansätze dar.

### Ergebnisse:

In der folgenden Tabelle 1 sind die ermittelten Kolonienzahlen des Testversuchs, die mit S. epidermidis ATCC 14 990 erhalten wurden, aufgelistet.

**Tabelle 1**

| A | 0,8 % Nanosilber, 0,5 % Silbersulfat, | | | | | | |
|---|---|---|---|---|---|---|---|
| | | | | | | | |
| Zeit in Stunden | | 0 | 6 | 12 | 24 | 36 | 48 |
| 5 x 10⁷ CF/ml | | | | | | | |
| 1 cm^{2*} | | 5 x 10⁷ | 2 x 10³ | 10³ | 0 | 0 | - |
| 5 cm² | | 5 x 10⁷ | 10³ | 0 | 0 | 0 | - |
| | | | | | | | |

| 10⁹ CFU | | | | | | | |
|---|---|---|---|---|---|---|---|
| 1 cm² | | 10⁹ | 10⁷ | 0 | 0 | 0 | - |
| 5 cm² | | 10⁹ | 10⁵ | 0 | 0 | 0 | - |
| | | | | | | | |

| B | 0,8 % Nanosilber, 1,0 % Silbersulfat | | | | | | |
|---|---|---|---|---|---|---|---|
| | | | | | | | |
| Zeit in Stunden | | 0 | 6 | 12 | 24 | 36 | 48 |
| 5 x 10⁷ CFU/ml | | | | | | | |
| 1 cm² | | 5 x 10⁷ | 10⁴ | 0 | 0 | 0 | - |
| 5 cm² | | 5 x 10⁷ | 10³ | 0 | 0 | 0 | - |
| | | | | | | | |

| 10⁹ CFU | | | | | | | |
|---|---|---|---|---|---|---|---|
| 1 cm² | | 10⁹ | 10⁶ | 10² | 0 | 0 | - |
| 5 cm ^{2**} | | 10⁹ | 10⁴ | 0 | 0 | 0 | - |
| | | | | | | | |

| C | 0,8 Nanosilber (handesüblicher Kunststoff gemäß WO 01 /09229; Fa. Medex) | | | | | | |
|---|---|---|---|---|---|---|---|
| | | | | | | | |
| Zeit in Stunden | | 0 | 6 | 12 | 24 | 36 | 48 |
| 5 x 10⁷ CFU/ml | | | | | | | |
| 1 cm² | | 5 x 10⁷ | 10⁷ | 10⁶ | 10⁴ | 10³ | 0 |
| 5 cm² | | 5 x 10⁷ | 10⁶ | 10⁵ | 10³ | 10² | 0 |
| | | | | | | | |

| 10⁹ CFU | | | | | | | |
|---|---|---|---|---|---|---|---|
| 1 cm² | | 10⁹ | 10⁹ | 10⁹ | 10⁹ | 10⁹ | 10⁸⁺ |
| 5 cm² *** | | 10⁹ | 10⁹ | 10⁹ | 10⁹ | 10⁹ | 10⁶⁺ |

| | | | | | | | |
|---|---|---|---|---|---|---|---|
| * schwaches Wachstum der Kolonien nach 48 Stunden Bebrütung * in Figur 1 abgebildet ** in Figur 2 abgebildet *** in Figur 3 abgebildet | | | | | | | |

Ein entsprechendes Wachstumsverhalten zeigten auch der Wildstamm von S. epidermidis, S. aureus ATCC 25923 sowie E. coli und P. aeruginosa. Die Testversuche zeigten, dass die Zugabe von Silbersulfat die sofortige antimikrobielle Wirksamkeit deutlich steigert (Vergleich von A oder B gegenüber C). Die Steigerung der Wirksamkeit durch Zugabe von Silbersulfat ist dosisabhängig, jedoch kann bereits bei der Zugabe von 0,5 % Silbersulfat eine Wirksamkeit beobachtet werden. Der erfindungsgemäße Kunststoff zeigt eine deutlich verbesserte antimikrobielle Wirksamkeit im Vergleich zu einem Kunststoff, der lediglich Nanosilber enthält (Ansatz C). Bei dem getesteten Kunststoff des Standes der Technik (gemäß WO 01/09229) kann bei einer Ausgangskeimzahl von 5 x 10⁷ CFU/ml erst nach 48 Stunden eine Sterilität beobachtet werden. Bei einer Ausgangskeimzahl von 10⁹ CFU/ml tritt selbst nach 48 Stunden noch ein schwaches Wachstum der Kolonien auf.

### Beispiel 5

### Untersuchung von Zirkonsilikat-haltigem Trägermaterial

Das Trägermaterial Bariumsulfat wird in einer ersten Versuchsreihe mit 20 Gew.-% Zirkonsilikat, in einer zweiten Versuchsreihe mit 20 Gew.-% Nanosilber und 20 Gew.-% Zirkonsilikat versetzt. Die so erhaltenen Mischungen werden mit unterschiedlichen Mengen an Keimen versetzt und dann das Keimwachstum über 48 Stunden aufgezeichnet.

| Zeit | Zirkonsilikat ohne Silber Keimzahl/ml | | | | Zirkonsilikat mit Nanosilber Keimzahl/ml | | | |
|---|---|---|---|---|---|---|---|---|
| 0 | 10⁹ | 10⁸ | 10⁷ | 10⁶ | 10⁹ | 10⁸ | 10⁷ | 10⁶ |
| 2 h | + | + | + | - | + | +/- | - | - |
| 3 h | + | + | + | - | + | +/- | - | - |
| 6 h | + | + | +/- | - | + | - | - | - |
| 12 h | + | + /- | - | - | + | - | - | - |
| 18 h | + | + /- | - | - | + | - | - | - |
| 24 h | + | - | - | - | + | - | - | - |
| 30 h | + | - | - | - | + | - | - | - |
| 36 h | + | - | - | - | + | - | - | - |
| 42 h | + | - | - | - | +/- | - | - | - |
| 48 h | + | - | - | - | - | - | - | - |

| | | | | | | | | |
|---|---|---|---|---|---|---|---|---|
| + = Wachstum - = steril +/- = kein Wachstum aber auch noch nicht steril | | | | | | | | |

### Beispiel 6

### Vergleichende Untersuchung der antimikrobiellen Wirksamkeit von Zirkoniumsilikat auf Bariumsulfat als Träger alleine oder mit Nanosilber

| Zeit (Std). | 1 | 2 | 3 | 4 | 6 | 9 | 12 |
|---|---|---|---|---|---|---|---|
| Kontrolle (Keimzahl/ml) | 10⁹ | 10⁹ | 10⁹ | 10⁹ | 10⁹ | 10⁹ | 10⁹ |
| | | | | | | | |
| 1 % Zirkoniumsilikat auf Bariumsulfat | 10⁹ | 10⁸ | 10⁸ | 10⁷ | 10⁷ | 10⁶ | 10⁵ |
| | | | | | | | |
| 0,1 % Zirkoniumsilikat auf Bariumsulfat | 10⁹ | 10⁹ | 10⁹ | 10⁹ | 10⁹ | 10⁹ | 10⁸ |
| | | | | | | | |
| 1 % Zirkoniumsilikat + 5 % Nanosilber auf Bariumsulfat | 10⁸ | 10⁶ | - | - | - | - | - |
| | | | | | | | |
| 0,1 % Zirkoniumsilikat + 5 % Nanosilber auf Bariumsulfat | 10⁹ | 10⁷ | 10⁵ | - | - | - | - |
| | | | | | | | |
| 1 % Zirkoniumsilikat und 3,5 % Nanosilber auf BaSO₄ | 10⁹ | 10⁸ | 10⁶ | - | - | - | - |
| | | | | | | | |
| 0,1 % Zirkoniumsilikat + 3,5 % Nanosilber auf Bariumsulfat | 10⁹ | 10⁹ | 10⁷ | 10⁶ | 10⁶ | 10⁵ | - |

### Beispiel 7

### Untersuchung der antimikrobiellen Wirksamkeit bei Verwendung von Nanosilber und Silberphosphat in statu nascendi auf Bariumsulfatträger (3,6 % Ag; 5 % Silberphosphat

Adsorption von kolloidalem Silber an Bariumsulfat und Erzeugung von feinstverteiltem Silberphosphat in statu nascendi
In 360 ml destilliertem Wasser werden bei 50 °C 14,45 g Silbernitrat gelöst und anschließend unter kräftigem Rühren 100 g Bariumsulfat eingetragen. Die Suspension wird ca. 20 Minuten gerührt. Danach werden 19,3 ml einer 25 %-igen Ammoniaklösung zugesetzt.

Unter ständigem Rühren und bei gleichbleibender Temperatur wird in die Suspension langsam eine Lösung aus 5,25 g Glucosemonohydrat in 182 ml destilliertem Wasser dosiert. Nach Beendigung der Glucosezugabe wird weiter 2 bis 4 Stunden bei 50 °C gerührt und schließlich mit ca. 50 ml einer 0,1 molaren Phosphorsäure das noch vorhandene nicht reduzierte Silber gefällt und die Suspension auf pH = ca. 6 gebracht.

Das Rühren wird bis zur Abkühlung auf Zimmertemperatur fortgesetzt. Anschließend erfolgt die Feststoffabtrennung durch Sedimentation, Filtration oder Zentrifugation.

Der erhaltene Feststoff wird bis zur Elektrolytfreiheit mehrfach mit Reinstwasser gewaschen und zuletzt bei 70 bis 80 °C im Trockenschrank getrocknet und gegebenenfalls nach dem Trocknen zerkleinert.

Das auf diese Weise hergestellte Produkt ist von weißgrauer Farbe. es hat die Zusammensetzung 3,6 % Nanosilber, 5 % Silberphosphat auf BaSO₄. Die Keimzahl bei einer Konzentration von 1 % bzw. 0,1 % wurde gemäß Beispiel 4 bestimmt:

| Zeit (Std) | 1 | 2 | 3 |
|---|---|---|---|
| 1 % | 10⁷ | 10⁵ | |
| 0,1 % | 10⁸ | 10⁷ | 10⁶ |

### Beispiel 8

### A: Adsorption von kolloidalem Silber an Bariumsulfat

In 360 ml auf 50 °C erwärmtem destilliertem Wasser werden 9 g Silbernitrat gelöst und unter kräftigem Rühren 100 g Bariumsulfat eingetragen. Nach 20 Minuten Rühren werden 12 ml einer 25 %-igen Ammoniaklösung zugesetzt.

Anschließend wird bei gleichbleibender Temperatur eine Lösung aus 5,25 g Glucosemonohydrat in 182 ml destilliertem Wasser langsam zudosiert. Nach Beendigung der Glucosedosierung wird die Suspension noch weitere 2 bis 4 Stunden bei 50 °C und anschließend noch 1 bis 3 Stunden bei 70 °C gerührt.

Nach der kompletten Reaktion wird der Feststoff aus der wässrigen Phase abgetrennt und bis zur Elektrolytfreiheit noch mehrmals mit Reinstwasser oder destilliertem Wasser gewaschen. Der gewaschene Feststoff wird bei 70 bis 80 °C im Trockenschrank getrocknet und danach auf die Primärkorngröße zerkleinert.

### B: Zumischen von Silberphosphat

Dem nach A erhaltenen Feststoff wird die gewünschte Menge (1 bis 5 Gew.-%) reinstes Silberphosphat zugesetzt und intensiv vermischt. Die Untersuchung wie in Beispiel 4 beschrieben ergab ebenso gute Resultate wie in Beispiel 7 gezeigt.

## Patentansprüche

1. Verfahren zur Herstellung eines antimikrobiellen Kunststoffproduktes, umfassend
A) Bilden eines Vorprodukts,
B) Behandeln von mindestens einem Bestandteil des Vorproduktes mit einem antimikrobiellen kolloiden Metall,
C) Zusetzen eines löslichen oder schwerlöslichen Salzes eines antimikrobiellen Metalls und
D) Formen des Kunststoffproduktes.

2. Verfahren nach Anspruch 1, **dadurch gekennzeichnet, daß** das schwerlösliche Metallsalz ausgewählt ist aus der Gruppe, bestehend aus Silbersalzen, Zinksalzen, Kupfersalzen, Cersalzen, Zirkoniumsalzen, Wismutsalzen, Platinsalzen oder/und Goldsalzen.

3. Verfahren nach Anspruch 2, **dadurch gekennzeichnet, daß** das Metallsalz Silbersulfat oder/und Silberphosphat umfaßt.

4. Verfahren nach Anspruch 3, **dadurch gekennzeichnet, daß** das Metallsalz in der Menge von 0,1 bis 1,0 Gew.-% bezogen auf das Gesamtgewicht des Vorprodukts vorliegt.

5. Verfahren nach Anspruch 2, **dadurch gekennzeichnet, daß** das Metallsalz in einem Silber/Kupfer-Verhältnis von etwa 2:1 vorliegt.

6. Verfahren nach einem der Ansprüche 1 bis 5, **dadurch gekennzeichnet, daß** das Vorprodukt ein oder mehrere polymere Materialien umfaßt.

7. Verfahren nach Anspruch 6, **dadurch gekennzeichnet, daß** das Vorprodukt Polyurethan umfaßt.

8. Verfahren nach einem der Ansprüche 1 bis 7,**dadurch gekennzeichnet, daß** das Vorprodukt weitere Additive umfaßt.

9. Verfahren nach Anspruch 8. **dadurch gekennzeichnet, daß** die Additive anorganische oder organische Teilchen umfassen.

10. Verfahren nach Anspruch 9, **dadurch gekennzeichnet, daß** die anorganischen oder/und organischen Teilchen ausgewählt sind aus der Gruppe, bestehend aus Bariumsulfat, Calciumsulfat, Strontiumsulfat, Titandioxid, Aluminiumoxid, Siliciumdioxid, Zeolithe, Calciumfluorid (CaF₂), Glimmer, Talk, pyrogene Kieselsäure, Calciumhydroxylapatit, Kaolin. Zirkon oder/und Mikrocellulose.

11. Verfahren nach Anspruch 10, **dadurch gekennzeichnet, daß** die anorganischen Teilchen Bariumsulfat oder/und pyrogene Kieselsäure umfassen.

12. Verfahren nach einem der Ansprüche 1 bis 11, **dadurch gekennzeichnet, daß** polymere Materialien und anorganische Teilchen mit einem kolloiden Metall behandelt werden.

13. Verfahren nach einem der Ansprüche 1 bis 11, **dadurch gekennzeichnet, daß** anorganische Teilchen mit einem kolloiden Metall behandelt werden.

14. Verfahren nach einem der vorhergehenden Ansprüche, **dadurch gekennzeichnet, daß** das kolloide Metall kolloidales Silber umfaßt.

15. Verfahren nach einem der vorhergehenden Ansprüche, **dadurch gekennzeichnet, daß** das Gemisch aus behandeltem Vorprodukt und schwerlöslichem Metallsalz durch Extrudieren, Spritzgießen, Mischen, Kneten oder (Heiß-) Pressen geformt wird.

16. Kunststoffprodukte, erhältlich nach einem der Ansprüche 1 bis 15.

17. Kunststoffprodukt nach Anspruch 16 in Form eines Katheters.

## Claims

1. Method for producing an antimicrobial plastics material product, comprising
A) forming an intermediate product,
B) treating at least one constituent of the intermediate product with an antimicrobial colloidal metal,
C) adding a soluble or poorly soluble salt of an antimicrobial metal and
D) forming the plastics material product.

2. Method according to claim 1, **characterised in that** the poorly soluble metal salt is selected from the group consisting of silver salts, zinc salts, copper salts, cerium salts, zirconium salts, bismuth salts, platinum salts and/or gold salts.

3. Method according to claim 2, **characterised in that** the metal salt comprises silver sulphate and/or silver phosphate.

4. Method according to claim 3, **characterised in that** the metal salt is present in the quantity of 0.1 to 1.0 % by weight based on the total weight of the intermediate product.

5. Method according to claim 2, **characterised in that** the metal salt is present in a silver/copper ratio of about 2:1.

6. Method according to any one of claims 1 to 5, **characterised in that** the intermediate product comprises one or more polymeric materials.

7. Method according to claim 6, **characterised in that** the intermediate product comprises polyurethane.

8. Method according to any one of claims 1 to 7, **characterised in that** the intermediate product comprises further additives.

9. Method according to claim 8, **characterised in that** the additives comprise inorganic or organic particles.

10. Method according to claim 9, **characterised in that** the inorganic or/and organic particles are selected from the group consisting of barium sulphate, calcium sulphate, strontium sulphate, titanium dioxide, aluminium oxide, silicon dioxide, zeolites, calcium fluoride (CaF₂), mica, talc, pyrogenic silica, calcium hydroxylapatite, kaolin, zircon or/and microcellulose.

11. Method according to claim 10, **characterised in that** the inorganic particles comprise barium sulphate or/and pyrogenic silica.

12. Method according to any one of claims 1 to 11, **characterised in that** polymeric materials and inorganic particles are treated with a colloidal metal.

13. Method according to any one of claims 1 to 11, **characterised in that** inorganic particles are treated with a colloidal metal.

14. Method according to any one of the preceding claims, **characterised in that** the colloidal metal comprises colloidal silver.

15. Method according to any one of the preceding claims, **characterised in that** the mixture of treated intermediate product and poorly soluble metal salt is formed by extruding, injection moulding, mixing, kneading or (hot) pressing.

16. Plastics material products, obtainable according to any one of claims 1 to 15.

17. Plastics material product according to claim 16 in the form of a catheter.

## Revendications

1. Procédé de préparation d'un produit antimicrobien en matière plastique, comprenant
A) la formation d'un(e) ébauche ou produit semi-fini,
B) le traitement d'au moins un constituant de l'ébauche ou du produit semi-fini avec un métal colloïdal antimicrobien,
C) l'addition d'un sel soluble ou difficilement soluble d'un métal antimicrobien, et
D) la formation du produit de matière plastique.

2. Procédé selon la revendication 1, **caractérisé en ce que** le sel métallique difficilement soluble est choisi parmi le groupe consistant en des sels d'argent, des sels de zinc, des sels de cuivre, des sels de cérium, des sels de zirconium, des sels de bismuth, des sels de platine et/ou des sels d'or.

3. Procédé selon la revendication 2, **caractérisé en ce que** le sel métallique comprend le sulfate d'argent et/ou le phosphate d'argent.

4. Procédé selon la revendication 3, **caractérisé en ce que** le sel métallique est présent en une quantité allant de 0,1 à 1,0 % en poids sur base du poids total de l'ébauche.

5. Procédé selon la revendication 2, **caractérisé en ce que** le sel métallique est présent en un rapport argent/cuivre d'environ 2:1.

6. Procédé selon l'une quelconque des revendications 1 à 5, **caractérisé en ce que** l'ébauche comprend un ou plusieurs matériaux polymères.

7. Procédé selon la revendication 6, **caractérisé en ce que** l'ébauche comprend du polyuréthanne.

8. Procédé selon l'une quelconque des revendications 1 à 7, **caractérisé en ce que** l'ébauche comprend d'autres additifs.

9. Procédé selon la revendication 8, **caractérisé en ce que** les additifs comprennent des particules inorganiques ou organiques.

10. Procédé selon la revendication 9, **caractérisé en ce que** les particules inorganiques et/ou organiques sont choisies parmi le groupe consistant en le sulfate de baryum, le sulfate de calcium, le sulfate de strontium, le dioxyde de titane, l'oxyde d'aluminium, le dioxyde de silicium, une zéolite, le fluorure de calcium (CaF₂), le mica, le talc, l'acide silicique pyrogène, l'hydroxylapatite de calcium, le kaolin, la zircone et/ou la microcellulose.

11. Procédé selon la revendication 10, **caractérisé en ce que** les particules inorganiques comprennent le sulfate de baryum et/ou l'acide silicique pyrogène.

12. Procédé selon l'une quelconque des revendications 1 à 11, **caractérisé en ce que** les matériaux polymères et les particules inorganiques sont traités avec un métal colloïdal.

13. Procédé selon l'une quelconque des revendications 1 à 11, **caractérisé en ce que** les particules inorganiques sont traités avec un métal colloïdal.

14. Procédé selon l'une quelconque des revendications précédentes, **caractérisé en ce que** le métal colloïdal comprend l'argent colloïdal.

15. Procédé selon l'une quelconque des revendications précédentes, **caractérisé en ce que** le mélange de l'ébauche traitée et du sel métallique difficilement soluble est formé par extrusion, moulage par injection, mélange, malaxage ou pressage (à chaud).

16. Produit de matière plastique, obtenu selon l'une quelconque des revendications 1 à 15.

17. Produit de matière plastique selon la revendication 16 sous forme d'un cathéter.
